# EUROPEAN PATENT SPECIFICATION

(11) **EP 1 951 852 B1**
(45) Date of publication and mention of the grant of the patent: **13.04.2016**
(21) Application number: 06844035.3
(22) Date of filing: 19.10.2006
(51) Int. Cl.: C11C 3/04, C07C 67/08, C10L 1/02, C10L 1/19

(54) **AUTOMOTIVE FUELS AND FINE CHEMICALS FROM CRUDE TALL OIL**
KRAFTFAHRZEUG-BRENNSTOFF UND FEINCHEMIKALIEN AUS ROHTALLÖL
CARBURANTS AUTOMOBILES ET PRODUITS CHIMIQUES FINS A PARTIR DE TALLOL

(30) Priority: 26.10.2005 US 730031 P; 22.05.2006 US 802131 P
(43) Date of publication of application: 06.08.2008
(73) Proprietor: SunPine AB, 94122 Piteå (SE)
(72) Inventor: STIGSSON, Lars, S-133 36 Saltsjöbaden (SE)
(74) Representative: Awapatent AB
(86) International application number: PCT/SE2006/050414
(87) International publication number: WO 2007/050030

(56) References cited:
- WO-A1-00/40743
- WO-A1-95/25152
- GB-A- 1 219 885
- US-A- 2 356 988
- US-A- 2 395 284
- US-A- 2 485 744
- US-A- 2 487 000
- US-A- 2 640 823
- US-A- 3 859 270
- US-A1- 2005 102 891

## Description

### Technical field

The present invention relates to a method for manufacturing fatty acid alkyl esters from crude tall oil (CTO) and other fatty acid rich raw materials.

### Background

In times of high cost:for fossil fuels and greenhouse gases emission considerations the interest for producing automotive fuels and chemicals from "green sources" such as wood has increased.

From both an environmental and economical point of view it is desired to replace conventional automotive fuels from crude oil with automotive fuels from renewable resources. Tall oil is a renewable raw material originating from wood. The tall oil comprises organic compounds that can be converted to combustion engine fuels such as diesel fuel. In addition it is also possible to recover and valorise other valuable compounds in tall oil such as resin acids and sterols.

Tall oil is a major by-product of the alkaline kraft pulping process. The tall oil originates from the extractives in the wood raw material. In the pulping process rosin acids (RA) and fatty acids (FA), which occur as free acids or their esters, are saponified by the alkaline cooking liquor to their corresponding sodium salts. These salts, or soaps, along with neutral organic components, often called unsaponifiables, are dissolved and suspended in the spent cooking liquor (black liquor). This liquor is later concentrated and the soaps and neutrals are separated as tall oil soap skimmings. Many pulp mills are recovering this soap and after acidulation a crude tall oil (CTO) is obtained for export or upgrade at the mill.

The tall oil recovered from a softwood kraft mill typically consist of approximately 35-60% fatty acids, including oleic, linoleic, linolenic and palmitic acids, 15-55% rosin acids, including abietic, dehydroabietic and neoabietic acids and 5-35% unsaponifiable and neutral material including sterols' such as beta-sitosterol. Hardwoods also contain extractives including fatty acids and neutrals(beta-sitosterol, betulin) but no resin acids.

In addition tall oil contains a small fraction of contaminants from black liquor such as sulphur compounds (up to 1000 ppm as S), lignin components and fibers. The tall oil is normally exported from the pulp mill to central tall oil distillation plants.

The production of alternative green biofuels such as biodiesel has experienced a strong growth over the past ten years. Biodiesel is normally produced from vegetable oils over catalytic transesterification to yield fatty acid alkyl esters (FAAE) which esters may be used partly or fully as a component in biodiesel fuel. In addition to replacing fossil based oil raw material, fatty acid alkyl esters are efficient lubricants in low sulphur diesel fuel.

Normally tall oil fatty acids comprise a large portion of linoleic acids providing for good cold flow properties of a biodiesel. Oxidative stability may be a problem due to the allylic and bisallylic double bonds in linoleic and linolenic acids respectively. Another concern with tall oil as raw material for biodiesel is the rather high content of sulphur compounds in tall oil (500-1000 ppm). The maximum allowable content of sulphur in biodiesel according to European and US standards for biodiesel is 10 ppm.

Over the years many processes have been suggested for recovery of valuable products from tall oil. Relevant prior art patent' and patent applications are given below.

US 4,992,605 discloses a process for producing a diesel fuel additive comprising treatment of a fatty acid with gaseous hydrogen under high pressure.

US 5,705,722 describes a process for production of a diesel fuel cetane number improver comprising contacting tall oil with hydrogen at high temperature.

US 2,640,823 discloses a process for treating tall oil, wherein free fatty acids selectively are esterified with a lower alcohol. The resultant mixture is extracted with a selective polar solvent to separate rosin acids and the esters of fatty acids and unsaponifiable matter. The raffinate is distilled to obtain fatty acids in purified form. Also other components in the tall oil are extracted and recovered.

US 2,294,446 discloses a process for treatment of tall oil comprising addition of a small amount of for instance sulphuric acid. It is described that sulphonated products resulting from the acid treatment act as catalysts. There is shown a step with centrifugal separation of the mixture. The mixture is then esterified using a lower alcohol such as methanol.

WO 2004/080942 discloses a process for obtaining fatty acid alkyl esters, rosin acids and sterols from tall oil. The tall oil is esterified with a lower alcohol, and the sterols are esterified with boric acid or transesterified with a catalyst. The fatty acid esters and the rosin esters are separated from the sterol esters. The fatty acid esters and the rosin acids are also separated. Esterification can be performed under acidic conditions for example using methane sulphonic acid as catalyst.

WO 2004/074233 discloses a process of treating crude tall oil (CTO) wherein the CTO is subjected to the steps; a) reacting the free fatty acids in the CTO with lower alcohols, b) separating the fatty acid alkyl esters from the remaining CTO to produce a first stream of fatty acid ester. Several subsequent steps of recovering other components of the CTO are also disclosed.

GB 1264058 discloses a fuel composition comprising a small amount of tall oil fatty acid.

US 3,859,270 discloses a method of refining impure fatty acids and fatty acid blends by removing impurities including sulphur-containing impurities, boiling range of which is close and/or overlapping with the boiling range of the fatty acids and fatty acid blends.

US 2,395,284 discloses a process of separating an organic mixture containing one or more types of rosin acids, fatty acids, sterols, and other unsaponifiable organic material. with particular reference to tall oil.

Before the invention is disclosed and described in detail, it is to be understood that this invention is not limited to particular configurations, process steps and materials disclosed herein as such configurations, process steps and materials may vary somewhat. It is also to be understood that the terminology employed herein is used for the purpose of describing particular embodiments only and is not intended to be limiting since the scope of the present invention is limited only by the appended claims and equivalents thereof.

It should be noted that, as used in this specification and the appended claims, the singular forms "a", "an" and "the" include plural referents unless the context clearly dictates otherwise.

### Summary of the present invention

The main objective of the present invention is to recover and upgrade crude tall oil to high value fine chemicals and automotive fuels. It is furthermore an objective to provide an esterification process with a higher yield of fatty acid alkyl ester than prior art. Yet another objective is to provide a method for producing an automotive biodiesel type of fuel with low sulphur content. A further objective is to provide a method for recovering fine chemicals from tall oil by selective esterification of fatty acids. Moreover there is disclosed a fatty acid alkyl ester, and a resin acid manufactured with the method according to the present invention. There is also disclosed a fuel composition comprising the fatty acid alkyl ester manufactured by the method of the present invention.

The present invention discloses an innovative sequence of reaction and separation steps enabling the production of a fatty acid alkyl ester from tall oil in high yield and with very low sulphur content. Furthermore other valuable fine chemicals such as resin acids and beta-sitosterol can be recovered in the sequential procedure described by the present invention. By continuous removal of water formed during esterification the yield of fatty acid alkyl ester is increased. Low sulphur content fatty acid alkyl ester can advantageously be used as a biodiesel fuel component and by-products from the reaction can be upgraded to valuable fine chemicals. Addition of raw materials comprising monounsaturated/saturated fatty acids to the esterification reactor feed stream will increase the oxidative stability of a CTO based fatty acid alkyl ester/biodiesel.

The present invention thus provides a method for manufacturing fatty acid alkyl esters from tall oil comprising the steps of: a) esterifying tall oil in at least one esterification reactor in the presence of an acidic catalyst'and a C1 to C8 alcohol to form a crude product stream comprising fatty acid alkyl esters and H₂O, and b) separating H₂O and alcohol from the crude product stream formed in step a) to form a dehydrated fatty acid alkyl ester product stream, and c) separating dehydrated fatty acid alkyl ester products tream from step b) into at least two product streams wherein one stream is enriched in fatty acid alkyl esters and one stream is enriched in resin acid compounds.

Further embodiments of the present invention are described in the following description and appended dependent claims.

### Description of the drawing

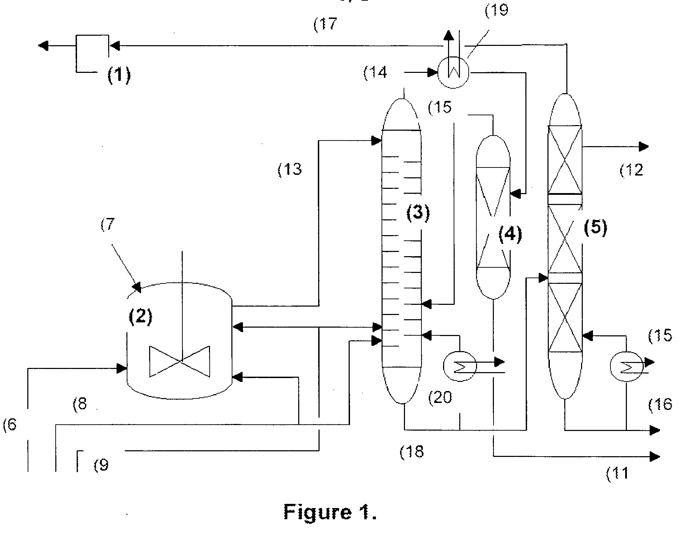

Figure 1 shows one embodiment of the esterification method of the present invention.

In the particular embodiment described in figure 1 dehydrated and heat-treated tall oil (by which heat treatment volatile sulphur compounds is removed) is transferred through line 6 to a continuously operating stirred tank reactor CSTR (2). Palm fatty acid distillate comprising at least 70 % free fatty acids is fed through line (7) to the CSTR (2). The proportion of palm fatty acids feed and tall oil feed to the CSTR is 1 to 1. Dry methanol is injected into the CSTR (2) through line (8). An acidic esterification catalyst PTSA (para toluene sulphonic acid) corresponding to a weight of 0,5 % of total amount of fatty acids fed to the CSTR is injected to the CSTR (2) through line (9). A reaction mixture comprising fatty acid methyl ester and unreacted fatty acids, resin acids, methanol and H₂O is discharged from the CSTR (2) to a reactive distillation column (3) through line (13). Substantially all of the fatty acids fed to the reactive distillation column (3) through line (13) are converted to fatty acid methyl ester during the downward passage of fatty material in the reactive distillation column. Esterification catalyst is added to the reactive distillation column through line (9). The temperature and pressure in the reactive distillation column (3) is selected so that unreacted methanol and H₂O is evaporated and discharged from the column through line (14). The steam (H₂O) and methanol mixture leaving the column through line (14) is cooled in cooler (19) and charged to a methanol stripper (4) wherein H₂O and methanol is separated into two streams. Methanol leaving the stripper (4) through line (15) is recycled and injected in gaseous form to the reactive distillation column (3). H₂O is leaving the methanol stripper (4) through line (11). Volatile sulphur compounds present in gaseous stream (14) can be removed by adsorption, fractionation or alkali scrubbing (not shown).

The crude fatty acid methyl ester rich product stream leaving the reactive distillation column (3) is transferred through line (18) to an evaporation/distillation column (5) operating under vacuum (0,05 bar). A portion of the crude fatty acid methyl ester is recycled to the reactive distillation column after heating in heat exchanger (20). In the evaporation/distillation column (5) the crude fatty acid methyl ester rich product is divided in two final product streams. One product stream comprising substantially pure fatty acid methyl ester (FAME) is discharged from the upper part of the evaporation/distillation column through line (12). The FAME is further purified and exported for blending into a biodiesel automotive fuel. The second product stream comprising resin acids and neutral high boiling components is discharged from the bottom part of the evaporation/distillation column through line (16). Part of the bottom fraction is recycled and preheated in heater (15) before re-injection in the evaporation/distillation column (5). The resin acid and neutral component rich stream (16) can be used directly as a biofuel or be treated for recovery of pure resin acids and beta-sitosterol. Vacuum is provided for in the column (5) by line (17) connected to a vacuum pump system (1).

A small portion of undesired sulphur compounds are volatilised in the evaporation column (5) and removed through the vacuum system line (17). Any undesired sulphur left in the fatty acid alkyl ester product discharged through line 12 is removed by a caustic treatment (NaOH) reducing the total sulphur content of the product fatty acid alkyl ester to below 10 ppm.

It is to be understood that this invention is not limited to the particular embodiment shown above. The scope of the present invention is limited only by the appended claims and equivalents thereof.

### Detailed description of the invention

The inventor of the present invention has discovered a new and efficient method for the production of pure fatty acid alkyl esters (fatty acid alkyl esters) in high yield from fatty acids present in tall oil. In addition a valuable by-product stream comprising resin acids is also recovered. Optionally a stream of neutral components may be recovered as yet another valuable by-product stream.

The feedstock material of the present invention is tall oil originating from crude tall oil soap traditionally recovered in alkaline pulp mills. The crude tall oil soap comprises fatty acid and resin acid soaps, neutral organic components and a small portion of entrained black liquor components (lignin, sulphur compounds and fibres). As a first step a major portion of the entrained black liquor components in the crude tall oil soap could be removed by mechanical/physical separation using for example a decanter centrifuge. Mechanically/physically purified crude tall oil soap may thereafter be further purified in a step removing at least some of the neutral components by solvent extraction to form purified tall oil soap. Optionally the tall oil soap mixture is purified by solvent extraction prior to forming crude tall oil by lowering the pH of tall oil soap mixture by addition of acid.

Crude tall oil is traditionally produced in pulp mills by acidulation of tall oil soap with sulphuric acid. The tall oil is often dried in a dehydrator to form a substantially dry tall oil prior to export from the mill.

Crude tall oil (CTO) recovered in accordance with the procedures described above is a raw material feed for the esterification reactor or reactors of the present invention. Other fatty acid containing material may also be fed to the esterification reactor or reactors. The objective of esterification is to form fatty acid alkyl esters (fatty acid alkyl esters) in high yield. The esterification plant comprises at least one rector with a catalytic esterification stage wherein fatty acids present in the CTO are selectively esterified in the presence of a catalyst and a C1 to C8 alcohol such as methanol, ethanol or iso-propanol. Preferably the alcohol is methanol or ethanol.

The physical conditions in the reactor or reactors and the catalyst are preferably selected so that fatty acids in the tall oil are esterified in preference to resin acids. It is well known that fatty acids with primary carboxylic acid. groups are esterified at milder conditions relative to resin acids and this fact is exploited for example in analytic procedures to quantify the portion of fatty acids in tall oil.

A problem with fatty acid esterification is the formation of H₂O, which drives the equilibrium esterification reaction backwards. Therefore H₂O should be removed from the reaction mixture in order to obtain fatty acid alkyl esters in high yield. The initial step of a fatty acid esterification reaction is the protonation of the acid to give an oxonium ion, which undergoes an exchange reaction with an alcohol to give an intermediate reactant. The intermediate reactant in turn will lose a proton to become an ester. Each step in the process is reversible but in the presence of very large excess of the alcohol, the equilibrium of the reaction is displaced so that esterification proceeds virtually to completion. However, in the presence of H₂O, which is a stronger electron donor than are aliphatic alcohols, formation of the intermediate is not favoured and esterification will not proceed fully. Thus H₂O has to be removed from the reaction mixture to achieve a high esterification yield. The total yield of fatty acid alkyl ester calculated on fatty acids in the feed streams to the reactor or reactors is over 80 %, preferably over 90 % and may in some preferred embodiments be as high as 98 % provided that H₂O is continuously and efficiently removed from the reaction mixture. Alcohols with a lower boiling point than H₂O are in one embodiment removed together with the alcohol from the crude product stream during the esterification reaction. The H₂O and alcohol vapours are collected and separated into a H₂O rich stream and an alcohol rich stream. The alcohol rich stream is in a preferred embodiment recycled to the esterification reactor or reactors.

In one embodiment the sulphur compounds are separated from the crude product stream together with the separation of H₂O and alcohol.

The free fatty acids present in the CTO are precluding the use of alkaline catalysts for the esterification reaction. Alkaline catalysts are normally used in the state of the art production of biodiesel or fatty acid alkyl esters through transesterification of vegetable oils. Alkaline components will convert fatty acids into their soaps, which in turn will create emulsification problems. Formation of emulsions is undesirable by inhibiting mass transfer during esterification and lowers the reaction yield. Therefore the present invention discloses the use of an acidic catalyst which catalyst can be either homogeneous or heterogeneous.

While sulphuric acid or other strong mineral acids can be used as catalyst, organosulphonic acids are particularly suitable homogeneous acidic catalysts for use in the present invention. Spent acid catalyst that is not recycled within the process itself can conveniently be separated and recycled to a kraft pulp mill liquor cycle. Particularly preferred organosulphonic acids include para-toluene sulphonic acid and methane acid. Heterogeneous fatty acid esterification catalysts are known and in particular solid resinous catalysts including organo acid-functionalised mesoporous silica catalysts can be used in the practise of the present invention. Other solid acidic heterogeneous catalysts that can be used include sulphated zirconium (SZ), tungstated zirconium (WZ), commercially available esterification catalysts such as Amberlyst 15 (A-15) and Nafion supported on silica (SAC-13). The acid catalyst preferably comprises at least one compound selected from the group consisting of sulphuric acid, organic acids such as para-toluene sulphonic acid and methane acid, solid resinous catalysts based on methane sulphonic acids, organosulphonic acid-functionalised mesoporous silica, sulphated zirconium, tungstated zirconium and mixtures thereof.

Esterification reactions converting fatty acids in CTO in accordance with the procedures described above can be performed in batch or semi batch reactors. However it is preferred to conduct at least a portion of the esterification reactions in continuous reactors such as continuous stirred tank reactors (CSTR). A particularly preferred reactor system for performing the esterification reactions is based on reactive distillation technology alone or in combination with at least one CSTR. An arrangement for refluxing excess alcohol can be used as an alternative to reactive distillation. In a preferred embodiment of the present invention the esterification reactions are performed using at least one continuous stirred tank reactor (CSTR) and at least one reactive distillation reactor in series.

Reactive distillation combines chemical reaction and distillation in one vessel. The combination of reaction and separation in one piece of equipment offers distinct advantages over conventional, sequential approaches. Especially for equilibrium limited reactions such as esterification and ester hydrolysis reactions, conversion can be increased far beyond chemical equilibrium conversion due to the continuous removal of reaction products from the reaction zone.

Because of complex interactions between chemical reaction and separation, the performance of a reactive distillation column is influenced by several parameters, e.g. size and location of reactive and non-reactive column sections, reflux ratio, feed location, or throughput. A pre-reactor, for example a CSTR, may be installed to mix the reactants CTO and alcohol (optionally also catalyst) prior to charging the reactants to the reactive distillation column. The reaction temperature in the pre-reactor and reactive distillation column is selected for achieving optimum conversion of the fatty acids present in the CTO into alkyl esters minimizing the conversion of the more stable resin acids to esters. Such temperature, normally in the range from about 50 to about 250°C is also selected to minimize undesired side reactions such as dimerisation of the fattv acids. The pressure in the reactive distillation column is from 1 - 50 bars, preferably in the range from 3-30 bars. Thus the tall oil esterification reactions are preferably carried out at a temperature in the range from about 50 to about 250°C, preferably in the range from 65 to 140°C.

When a homogeneous catalyst is used to catalyze the esterification reactions the column should preferably have structured packing internals. When a heterogeneous catalyst is used the catalyst is preferably immobilised by a column packing structure such as for example Katapak by Sulzer Chemtec.

Certain alcohols in the range C2-C8 including ethanol form azeotropes with H₂O which complicates the recycling of H₂O-free alcohol to the esterification reaction zone. In applications using an azeotrope forming alcohol as a reactant, entrainers can be added to be present during the esterification reaction. In one embodiment an entrainer is added to the reaction mixture to promote separation of excess alcohol and H₂O. Entrainers are well known for use as supporting additives in the art of azeotropic distillation. Without entrainer only the temperature and pressure could change the physical properties of the CTO alcohol mixture in the reactive distillation column. Changing temperature and/or pressure is not always possible, in particular for non-ideal mixtures such as CTO and alcohol. Furthermore such changes may be incompatible with the reaction requirements. An important feature is that a reactive distillation process can be designed such that recycling of entrainer is realised internally, and not externally, as in conventional distillation processes. Suitable entrainer chemicals that has the appropriate solubilitv and azeotropic forming characteristics for use in the present CTO esterification process, includes alkanes such as hexane, alkenes such as 1-hexene and other organic compounds such as cyclohexadiene, propyl acetate, pentanone and di-propyl ether.

Neutral components present in the CTO feed do not react with alcohol under the conditions selected and neutrals are discharged from the esterification reactor or reactors with the high boiling temperature fraction (fatty acid alkyl ester /resin acid mixture). Spent homogeneous catalyst may also be present in discharged high boiling fraction.

A stoicheiometric surplus charge of alcohol is normally used to drive the conversion of CTO to fatty acid alkyl esters to completion. The stoicheiometric ratio of alcohol to fatty acids present in the feed is in the order of 1,1:1 to 3:1. Low boiling alcohol that has not reacted with the CTO will pass upwards as a gas together with H₂O vapour through a reactive distillation column. A refluxing section is optionally installed in the upper part of the column. Unreacted alcohol can be separated from H₂O outside a reactive distillation column by pervaporation or stripping, such recovered alcohol is preferably recycled to a reactive distillation column or to a pre-reactor to the column. The point of charge for CTO, homogeneous catalyst and alcohol have to be selected for the specific alcohol, catalyst and CTO composition and such optimum charging points can easily be determined by the artisan skilled in the art.

In cases where no pre-reactor is used a major portion of the alcohol is charged in gaseous form to the lower section of a reaction distillation column. The CTO and catalyst (if homogeneously catalysed process) is preferably charged to the upper section of the column and the reactants are thus passing counter currently with the gaseous alcohol through the column. A small portion of the alcohol may be entrained with the high boiling fatty acid alkyl ester /resin acid fraction to a sump recycler in the lower section of column. The presence of alcohol in the lower part of the column is advantageous in minimizing undesired side reactions.

Steam and excess alcohol is continuously evaporated or distilled off the esterification reaction mixture. Alcohol is recovered from the steam/alcohol mixture by stripping or pervaporation and is recycled to the alcohol storage or directly to the esterification reactor/reactors. The crude product stream rich in fatty acid alkyl esters also comprises resin acids and a portion of high boiling neutral components including sterols and squalene.

A sufficiently pure stream of fatty acid alkyl esters is obtained by taking advantage of the considerably higher vapour pressure and lower boiling point of the fatty acid alkyl esters relative to resin acids and neutrals. Typically, a distillation column, a short path evaporator or any other type of efficient evaporator operated under vacuum can be used to separate the fatty acid alkyl esters from the higher boiling resin acids and neutrals. The vacuum pressure should be selected considering the feed tall oil composition, particularly fatty/resin acid ratio, and should be in the range of 1 bar down to 0,0005 bar, preferably 0,8 to 0,005 bar. During this unit operation of the process also undesired organosulphur compounds can be removed thereby forming a third process stream. This third process stream rich in sulphur is discharged from the distillation or evaporator separately from the fatty acid alkyl ester product stream. In one embodiment a third stream enriched in volatile sulphur compounds is separated from said crude product stream. Said separation is in one embodiment accomplished by utilising the difference in vapour pressure between sulphur compounds and fatty acid alkyl ester and resin acids.

While fatty acid alkyl esters are a main product from process, also the resin acids/ neutral component mixture can be valorised and used for various purposes. The resin acids/ neutral mixture can advantageously be further fractionated for recovery of fine chemicals. The neutrals can be separated from the resin acids by an aqueous alkaline wash dissolving the resin acids in the form of soaps. Neutrals are not dissolved in aqueous alkaline solutions. Additives such as polyelectrolytes and surfactants may be added to prevent emulsification, formation of micelles and other colloid structures disturbing the separation of neutrals and resin acid soaps in two phases. The resin acids can thereafter be obtained in reasonably pure form by acidulating the aqueous soap stream. Due to the high melting points of most resin acids the acidulation should be performed at a temperature above 70°C. The neutral rich stream can be removed and purified to obtain valuable fine chemicals. Non-limiting examples of components in the neutral rich stream include beta-sitosterol.

Reasonably pure resin acids may also be obtained by washing the product stream directly from the esterification stage with an aqueous alkaline washing liquid. The resin acids are solubilised in the washing liquid, while the fatty acid alkyl esters are combined with the neutrals to form a lipophilic phase. Again, as disclosed above, the resin acids can be obtained after acidulation of the alkaline washing liquid. Fatty acid alkyl esters can be purified from neutrals by evaporation, taking advantage of the large difference in vapour pressure and boiling points of neutrals and fatty acid alkyl esters.

By the procedure described it is possible to manufacture three valuable products from the CTO; one stream rich in fatty acid alkyl esters, one stream rich in resin acids and one stream rich in neutral components.

The FAAE rich stream can be further treated by at least one method selected from purification, hydrogenation and dimerisation. FAAEs can be used as a component in biodiesel fuel or as a raw material in fine chemicals synthesises to surfactants and lubricants.

The current demand for fatty acid alkyl esters with very low sulphur content can be met by either stripping of organosulphur compounds from tall oil or reaction mixture streams as described above or by purification of the FAAE rich product stream. Examples of such methods include alkali treatment and selective sulphur adsorption. The sulphur of the fatty acid alkyl esters is thereby decreased to a level below about 300 ppm, preferably below about 50 ppm and most preferably to a level below about 10 ppm sulphur.

While the method for producing fatty acid alkyl esters in accordance with the description above is based on crude tall oil as fatty acid feedstock also other fatty acid rich raw materials are added and used as feed to the esterification reactor. Such fatty acid rich feedstocks include for example palm oil fatty acid distillates or other fatty acid rich materials containing oleic, palmitic or stearic acids. A large fraction of triglycerides, if present in such feedstocks, will be transesterified to fatty acid alkyl esters in the esterification reactors. According to the present invention a stream comprising at least one fatty acid in addition to tall oil is added to the esterification step. Said fatty acid is preferably at least one fatty acid selected from the group consisting of palm fatty acid distillate, stearic, palmitic or oleic acid. An advantage of feeding according to the present invention a saturated or monounsaturated fatty acid rich material such as palm fatty acids together with tall oil to the esterification reactor is that the oxidative stability of the product fatty acid alkyl esters can be improved. Furthermore, such addition of fatty acid rich feed to the esterification reactor or reactors enables the manufacturer to control the iodine number of the product fatty acid alkyl esters.

The procedure described herein for preparation of fatty acid alkyl esters can be combined with a crude tall oil soap neutrals purification process. Such processes are well known in the art and are often based on solvent extraction with the addition of demulsifiers to break crude tall oil soap H₂O emulsions. Besides the value of the neutral components as raw material for food ingredients and pharmaceuticals the purified tall oil material obtains a higher acid value. Acid value is a quality parameter for crude tall oil and a higher acid value tall oil feed is also advantageous in the method of the present invention. Neutral rich streams recovered from any of the process stages of the present invention can be further treated for example by evaporation, distillation or by crystallization to recover sterols and other organic fine chemicals originally present in the crude tall oil soap. In one embodiment the tall oil soap is purified in order to increase the acid value of tall oil and to recover neutral components present in the tall oil soap mixture.

In order to prevent undesired esterification reactions between fatty acids in tall oil on one hand and fatty alcohols and sterols also present in tall oil on the other hand, a C1-C8 alcohol can be added to the tall oil prior to performing the catalysed esterification step. Such addition has the advantage that an amount of the desired fatty acid alkyl ester is formed during for instance storage already before the catalysed esterification step is performed. Undesired esterification reactions between sterols and fatty acids are also inhibited.

In one embodiment volatile sulphur compounds are separated from tall oil by evaporation from tall oil prior to performing the esterification step.

The fatty acid alkyl ester product stream is in one embodiment, after the removal of H₂O and alcohol, divided into two separate process streams by evaporative separation at vacuum utilising the difference in boiling point between fatty acid alkyl esters and resin acids/neutral components.

In one embodiment a resin acid- and neutral component rich stream is separated into a resin acid rich stream and a neutral component rich stream by dissolution of resin acids as soaps in an alkaline aqueous mixture. In a preferred embodiment resin acids are recovered from resin acid soaps by treatment with an acid.

There is disclosed a biodiesel fuel composition comprising a fatty acid alkyl ester. Such fuel composition is either essentially pure
fatty acid alkyl ester or a blended biodiesel fuel composition comprising in addition to fatty acid alkyl ester also fossil fuels and other optional additives. Typical examples of such blended fuels are normally called B5 or B20 denoting the percentage of biodiesel in standard diesel fuels.

## Claims

1. A method for manufacturing fatty acid alkyl esters from tall oil containing sulphur compounds comprising the steps of:
a) esterifying tall oil in at least one esterification reactor in the presence of an acidic catalyst and a C1 to C8 alcohol, to form a crude product stream comprising fatty acid alkyl esters and H₂O,
b) separating H₂O and alcohol from the crude product stream formed in step a) to form a dehydrated fatty acid alkyl ester product stream,
c) separating dehydrated fatty acid alkyl ester product stream from step b) into at least two product streams wherein one product stream is enriched in fatty acid alkyl esters and one product stream is enriched in higher boiling point neutral and resin acid components, and wherein a material comprising a saturated or monounsaturated fatty acid is added together with the tall oil in step a).

2. The method according to claim 1, wherein the product stream enriched in fatty acid alkyl esters is further treated by at least one method selected from purification, hydrogenation, and dimerisation.

3. The method according to any one of claims 1 to 2 wherein at least a part of the alcohol obtained in step b) is recycled to the esterification system.

4. The method according to any one of claims 1 to 3, wherein volatile sulphur compounds are separated
i. from tall oil by evaporation from tall oil prior to esterification in step a), or
ii. from the crude product stream together with the separation of H₂O and alcohol in step b).

5. The method according to any one of claims 1 to 4 wherein a third stream enriched in sulphur compounds is separated during step c), said separation accomplished by utilising the difference in vapour pressure between sulphur compounds and fatty acid alkyl ester and resin acids

6. The method according to any one of claims 1 to 5, wherein an amount of a C1 to C8 alcohol is added to the tall oil prior to performing step a) of claim 1.

7. The method according to any one of claims 1 to 6 wherein tall oil charged to step a) of claim 1 is purified by solvent extraction of tall oil soap prior to forming said tall oil.

8. The method according to any one of claims 1 to 7, wherein the esterification of tall oil is performed using at least one continuous stirred tank reactor (CSTR) and/or at least one reactive distillation reactor and/or at least one reactor arrangement for refluxing excess alcohol.

9. The method according to any one of claims 1 to 8, wherein step a) is carried out at a temperature in the range from 50 to 250°C.

10. The method according to any one of claims 1 to 9, wherein step c) is carried out at a pressure in the range from 1 bar to 0,0005 bar.

11. The method according to any one of claims 1 to 10 wherein an entrainer is added to the reaction mixture to promote separation of excess alcohol and H₂O.

12. The method according to any one of claims 1 to 11, wherein the material comprising a saturated or monounsaturated fatty acid is at least one fatty acid selected from the group consisting of palm fatty acid distillate, stearic acid, palmitic acid and oleic acid.

13. The method according to any one of claims 1 to 12 wherein a resin acid- and neutral component rich stream is separated into a resin acid rich stream and a neutral component rich stream by dissolution of resin acids as soaps in an alkaline aqueous mixture.

14. The method according to claim 13 wherein resin acids are recovered from resin acid soaps by treatment with an acid.

15. A method for the manufacturing of a fuel composition, said method comprising the steps:
a) manufacturing of a fatty acid alkyl ester according to any one of claims 1-14,
b) adding of a fossil fuel,
c) optionally adding of at least one additive.

## Patentansprüche

1. Verfahren zur Herstellung von Fettsäurealkylestern aus Schwefelverbindungen enthaltendem Tallöl, das folgende Schritte umfasst:
a) Verestern von Tallöl in mindestens einem Veresterungsreaktor in Gegenwart eines sauren Katalysators und eines C1- bis C8-Alkohols zur Bildung eines Rohproduktstroms, der Fettsäurealkylester und H₂O umfasst,
b) Abtrennen von H₂O und Alkohol von dem in Schritt a) gebildeten Rohproduktstrom zur Bildung eines dehydratisierten Fettsäurealkylester-Produktstroms,
c) Auftrennen des dehydratisierten Fettsäurealkylester-Produktstroms aus Schritt b) in mindestens zwei Produktströme, wobei ein Produktstrom mit Fettsäurealkylestern angereichert ist und ein Produktstrom mit höhersiedenden Neutral- und Harzsäurekomponenten angereichert ist, und wobei in Schritt a) ein Material, das eine gesättigte oder einfach ungesättigte Fettsäure umfasst, zusammen mit dem Tallöl zugegeben wird.

2. Verfahren nach Anspruch 1, bei dem der mit Fettsäurealkylestern angereicherte Produktstrom durch mindestens ein aus Reinigung, Hydrierung und Dimerisierung ausgewähltes Verfahren weiterbehandelt wird.

3. Verfahren nach einem der Ansprüche 1 bis 2, bei dem mindestens ein Teil des in Schritt b) erhaltenen Alkohols in das Veresterungssystem zurückgeführt wird.

4. Verfahren nach einem der Ansprüche 1 bis 3, bei dem flüchtige Schwefelverbindungen
i. aus Tallöl durch Abdampfen von Tallöl vor der Veresterung in Schritt a) oder
ii. aus dem Rohproduktstrom zusammen mit der Abtrennung von H₂O und Alkohol in Schritt b) abgetrennt werden.

5. Verfahren nach einem der Ansprüche 1 bis 4, bei dem während Schritt c) ein dritter Strom, der mit Schwefelverbindungen angereichert ist, abgetrennt wird, wobei die Trennung durch Ausnutzung der Dampfdruckdifferenz zwischen Schwefelverbindungen und Fettsäurealkylester und Harzsäuren bewerkstelligt wird.

6. Verfahren nach einem der Ansprüche 1 bis 5, bei dem das Tallöl vor der Durchführung von Schritt a) von Anspruch 1 mit einer Menge eines C1- bis C8-Alkohols versetzt wird.

7. Verfahren nach einem der Ansprüche 1 bis 6, bei dem in Schritt a) von Anspruch 1 eingetragenes Tallöl durch Lösungsmittelextraktion von Tallölseife vor der Bildung des Tallöls gereinigt wird.

8. Verfahren nach einem der Ansprüche 1 bis 7, bei dem die Veresterung von Tallöl unter Verwendung mindestens eines kontinuierlich durchströmten Rührkessels (continuous stirred tank reactor, CSTR) und/oder mindestens eines Reaktivdestillationsreaktors und/oder mindestens einer Reaktoranordnung zum Refluxieren von überschüssigem Alkohol durchgeführt wird.

9. Verfahren nach einem der Ansprüche 1 bis 8, bei dem Schritt a) bei einer Temperatur im Bereich von 50 bis 250°C durchgeführt wird.

10. Verfahren nach einem der Ansprüche 1 bis 9, bei dem Schritt c) bei einem Druck im Bereich von 1 bar bis 0,0005 bar durchgeführt wird.

11. Verfahren nach einem der Ansprüche 1 bis 10, bei dem die Reaktionsmischung zur Förderung der Abtrennung von überschüssigem Alkohol und H₂O mit einem Schleppmittel versetzt wird.

12. Verfahren nach einem der Ansprüche 1 bis 11, bei dem es sich bei dem Material, das eine gesättigte oder einfach ungesättigte Fettsäure umfasst, um mindestens eine Fettsäure aus der Gruppe bestehend aus Palmfettsäuredestillat, Stearinsäure, Palmitinsäure und Ölsäure handelt.

13. Verfahren nach einem der Ansprüche 1 bis 12, bei dem ein harzsäure- und neutralkomponentenreicher Strom durch Lösen von Harzsäuren als Seifen in einer wässrig-alkalischen Mischung in einen harzsäurereichen Strom und einen neutralkomponentenreichen Strom aufgetrennt wird.

14. Verfahren nach Anspruch 13, bei dem Harzsäuren durch Behandlung mit einer Säure aus Harzsäureseifen zurückgewonnen werden.

15. Verfahren zur Herstellung einer Brennstoffzusammensetzung, wobei das Verfahren folgende Schritte umfasst:
a) Herstellen eines Fettsäurealkylesters nach einem der Ansprüche 1-14,
b) Zugeben eines fossilen Brennstoffs,
c) gegebenenfalls Zugeben mindestens eines Additivs.

## Revendications

1. Procédé de fabrication d'esters alkyliques d'acides gras à partir de tallöl contenant des composés soufrés comprenant les étapes consistant à :
a) estérifier du tallöl dans au moins un réacteur d'estérification en présence d'un catalyseur acide et d'un alcool en C1 à C8, pour former un courant de produits bruts comprenant des esters alkyliques d'acides gras et de l'H₂O,
b) séparer l'H₂O et l'alcool du courant de produits bruts formé à l'étape a) pour former un courant de produits esters alkyliques d'acides gras déshydratés,
c) séparer le courant de produits esters alkyliques d'acides gras déshydratés de l'étape b) en au moins deux courants de produits, un courant de produits étant enrichi en esters alkyliques d'acides gras et un courant de produits étant enrichi en composants neutres et acides résiniques à point d'ébullition supérieur, et dans lequel un matériau comprenant un acide gras saturé ou mono-insaturé est ajouté conjointement avec le tallöl à l'étape a).

2. Procédé selon la revendication 1, dans lequel le courant de produits enrichi en esters alkyliques d'acides gras est encore traité par au moins un procédé choisi parmi une purification, une hydrogénation, et une dimérisation.

3. Procédé selon l'une quelconque des revendications 1 et 2 dans lequel au moins une partie de l'alcool obtenu à l'étape b) est recyclée dans le système d'estérification.

4. Procédé selon l'une quelconque des revendications 1 à 3, dans lequel les composés soufrés volatils sont séparés
i. du tallöl par évaporation à partir du tallöl avant l'estérification à l'étape a), ou
ii. du courant de produits bruts conjointement avec la séparation de l'H₂O et de l'alcool à l'étape b).

5. Procédé selon l'une quelconque des revendications 1 à 4 dans lequel un troisième courant enrichi en composés soufrés est séparé pendant l'étape c), ladite séparation étant accomplie en utilisant la différence de pression de vapeur entre les composés soufrés et les esters alkyliques d'acides gras et les acides résiniques.

6. Procédé selon l'une quelconque des revendications 1 à 5, dans lequel une quantité d'un alcool en C1 à C8 est ajoutée au tallöl avant d'effectuer l'étape a) de la revendication 1.

7. Procédé selon l'une quelconque des revendications 1 à 6 dans lequel le tallöl chargé à l'étape a) de la revendication 1 est purifié par extraction par solvant de savon de tallöl avant de former ledit tallöl.

8. Procédé selon l'une quelconque des revendications 1 à 7, dans lequel l'estérification du tallöl est effectuée en utilisant au moins un réacteur à cuve agitée en continu (CSTR) et/ou au moins un réacteur de distillation réactive et/ou au moins un système de réacteurs pour porter au reflux l'alcool en excès.

9. Procédé selon l'une quelconque des revendications 1 à 8, dans lequel l'étape a) est réalisée à une température dans la fourchette de 50 à 250 °C.

10. Procédé selon l'une quelconque des revendications 1 à 9, dans lequel l'étape c) est réalisée à une pression dans la fourchette de 1 bar à 0,0005 bar.

11. Procédé selon l'une quelconque des revendications 1 à 10 dans lequel un entraîneur est ajouté au mélange réactionnel pour favoriser la séparation de l'alcool en excès et de l'H₂O.

12. Procédé selon l'une quelconque des revendications 1 à 11, dans lequel le matériau comprenant un acide gras saturé ou monoinsaturé est au moins un acide gras choisi dans le groupe constitué par le distillat d'acides gras de palme, l'acide stéarique, l'acide palmitique et l'acide oléique.

13. Procédé selon l'une quelconque des revendications 1 à 12 dans lequel un courant riche en acides résiniques et composants neutres est séparé en un courant riche en acides résiniques et un courant riche en composants neutres par dissolution des acides résiniques sous la forme de savons dans un mélange aqueux alcalin.

14. Procédé selon la revendication 13 dans lequel les acides résiniques sont récupérés à partir des savons d'acides résiniques par traitement avec un acide.

15. Procédé de fabrication d'une composition de carburant, ledit procédé comprenant les étapes suivantes :
a) fabriquer un ester alkylique d'acide gras selon l'une quelconque des revendications 1 à 14,
b) ajouter un carburant fossile,
c) ajouter éventuellement au moins un additif.
